# EUROPEAN PATENT APPLICATION

(11) **EP 2 865 763 A1**
(43) Date of publication of application: **29.04.2015**
(21) Application number: 13189659.9
(22) Date of filing: 22.10.2013
(51) Int. Cl.: C12Q 1/68

(54) **Method and kit for identifying and quantifying at least one single-stranded target nucleic acid**

(71) Applicant: Siemens Aktiengesellschaft, 80333 München (DE)
(72) Inventor: Battersby, Thomas, El Cerrito, CA 94530 (US); Gumbrecht, Walter, 91074 Herzogenaurach (DE); Hofmann, Stefan, 91056 Erlangen (DE); Huang,Yiwei, 91058 Erlangen (DE)
(74) Representative: Maier, Daniel Oliver

(57) **Abstract**

The invention concerns a method for identifying and quantifying at least one single-stranded target nucleic acid (13) and a kit for detecting at least one single-stranded target nucleic acid (13) in a sample. The method comprises providing at least one solid support (1) with at least one catcher oligonucleotide (5) immobilized on the support (1), contacting the support (1) with at least one first adapter oligonucleotide (7), at least one second adapter oligonucleotide (9) and at least one single-stranded target nucleic acid (13), the first and/or the second adapter oligonucleotide (7, 9) having a label, and the target nucleic acid (13) is identified by detecting the label of the first and/or the second adapter oligonucleotide (7, 9).

## Description

The invention concerns a method for identifying and quantifying at least one single-stranded target nucleic acid and a kit for detecting at least one single-stranded target nucleic acid in a sample.

The expression of genes in cells is strictly controlled and regulated by various molecular processes. In addition to the complex regulation of gene transcription, post-transcriptional regulation of gene expression, i. e. at the level of mRNA, plays an important role. In particular, small non-coding nucleic acids may interfere with gene expression by forming base pairs with complementary sequences within mRNA molecules, thereby affecting the translation of the mRNA in the corresponding protein. The short nucleic acids are often so-called microRNAs (abbreviated "miRNAs") that have gained attention in the fields of molecular biology and medicine during the last years. It was shown that some miRNAs are directly related to the development of certain cancer types such as breast cancer, lung cancer or leukaemia. The number of specific miRNAs is increased or decreased in cancer cells. Altered concentrations of miRNAs are also found in immunological diseases such as rheumatism. It is assumed that humans have more than 1000 different miRNA molecules.

Due to the increasing importance of miRNAs and their high number, methods for specific miRNA identification and quantification are needed. Different methods for detecting miRNAs are known, such as sequencing miRNAs or amplifying miRNAs by real-time PCR.

It is further known to identify miRNA by hybridizing the miRNA to a catcher oligonucleotide and/or a reporter oligonucleotide by complementary base pairing. In these cases, two component hybrids of miRNA and catcher/reporter oligonucleotides are formed. The problem of miRNA detection by two component hybridizations is that two component hybrids have low stability. Therefore, the hybrids may be resolved during stringent washing, resulting in a loss of label. Thus, the sensitivity of miRNA detection is very low.

Due to the low sensitivity of known detection methods, miRNAs often need to be amplified prior to their detection. During amplification, incorporation of incorrect nucleotides may occur, limiting the specificity of miRNA detection. Further, detection needs to be carried out at low temperatures due to low melting temperatures of miRNA hybrids that result from their short length. Therefore, miRNA detection is not very robust and cannot be quantitatively analyzed. In addition, many methods for miRNA identification require a pre-analytic labelling of the miRNA, bearing the risk of imprecise results and artefacts.

Therefore, a method for identifying and quantifying miRNA that overcomes these problems is needed, as well as a kit for performing the method.

The problem is solved by a method according to claim 1 and a kit according to claim 8.

The method according to the invention for identifying and quantifying at least one single-stranded target nucleic acid comprises the steps: providing at least one solid support with at least one catcher oligonucleotide immobilized on the support, the catcher oligonucleotide comprising a first portion and a second portion adjacent to each other, contacting the support at a first reaction condition with at least one first adapter oligonucleotide, at least one second adapter oligonucleotide and at least one single-stranded target nucleic acid, the target nucleic acid comprising a first portion and a second portion adjacent to each other, the first adapter oligonucleotide comprising an oligonucleotide sequence complementary to the first portion of the catcher oligonucleotide adjacent to an oligonucleotide sequence complementary to the first portion of the target nucleic acid, the second adapter oligonucleotide comprising an oligonucleotide sequence complementary to the second portion of the catcher oligonucleotide adjacent to an oligonucleotide sequence complementary to the second portion of the target nucleic acid, the first and/or the second adapter oligonucleotide having a label, the catcher oligonucleotide hybridizes with the respective complementary portions of the first and the second adapter oligonucleotide and the target nucleic acid hybridizes with the respective complementary portions of the first and the second adapter oligonucleotide such that a Holliday junction is formed. The steps to follow are: incubating the carrier at a second reaction condition such that the first and the second adapter oligonucleotide remain on the support only if the target nucleic acid is present, and detecting the label of the first and/or the second adapter oligonucleotide.

The term "target nucleic acid" refers to a single-stranded nucleic acid with a length of approximately 10 to approximately 30 nucleotides. The nucleic acid can be an RNA or a DNA. Naturally occurring nucleic acids as well as chemically produced or recombinantly produced nucleic acids are preferred target nucleic acids.

First, at least one solid support is provided for the method according to the invention. In a preferred embodiment, the solid support is a chip, preferably a silicon chip. Silicon chips can be produced at low costs and additional elements such as electrodes, temperature sensors and cooling and/or heating elements can easily be integrated on the chip.

In a more preferred embodiment, at least one electrode, preferably a gold electrode, is integrated on the surface of the support. Nucleic acids and oligonucleotides can easily be immobilized on gold surfaces. The electrode can be used for electrochemically detecting the label, such as by measuring a current.

In a preferred embodiment, the solid support has a temperature control and/or a temperature sensor for setting or measuring a temperature of the solid support. The solid support can further comprise cooling and/or heating elements for cooling and/or heating the support.

At least one catcher oligonucleotide is immobilized on the support. The term "oligonucleotide" refers to a nucleic acid. The term includes single-stranded as well as double-stranded nucleic acids. The term includes RNA molecules, so-called oligoribonucleotides, as well as DNA molecules, so-called oligodesoxyribonucleotides.

The catcher oligonucleotide comprises a first portion and a second portion adjacent to each other. The term "adjacent" means that two sequences directly follow each other on the oligonucleotide strand without a single nucleotide between them. Thus, the last nucleotide of the first portion of the catcher oligonucleotide is followed by the first nucleotide of the second portion of the catcher oligonucleotide.

The sequence of the first portion of the catcher oligonucleotide is complementary to a portion of the first adapter oligonucleotide. The sequence of the second portion of the catcher oligonucleotide is complementary to a portion of the second adapter oligonucleotide. The first and the second portion of the catcher oligonucleotide preferably have a length of up to approximately 15 nucleotides each.

In a preferred embodiment, the catcher oligonucleotide is a single-stranded catcher oligonucleotide.

In a preferred embodiment, the catcher oligonucleotide is an oligodesoxyribonucleotide.

In one embodiment, the catcher oligonucleotide is immobilized on the support via a thiol group.

In a preferred embodiment, the catcher oligonucleotide is immobilized on the support via a spacer. The spacer facilitates efficient hybridization of the catcher oligonucleotide with the respective complementary portions of the first and the second adapter oligonucleotide. The spacer is preferably made of thymidine nucleotides and can be immobilized on the support via a thiol group.

According to the method of the invention, the support is then contacted at a first reaction condition with at least one first adapter oligonucleotide, at least one second adapter oligonucleotide and at least one single-stranded target nucleic acid.

The target nucleic acid comprises a first portion and a second portion adjacent each other. The first and/or the second portion of the target nucleic acid preferably have a length of up to approximately 15 nucleotides each.

The first adapter oligonucleotide comprises an oligonucleotide sequence complementary to the first portion of the catcher oligonucleotide adjacent to an oligonucleotide sequence complementary of the first portion of the target nucleic acid. The oligonucleotide sequence complementary to the first portion of the catcher oligonucleotide preferably has a length of up to approximately 15 nucleotides. The oligonucleotide sequence complementary to the first portion of the target nucleic acid preferably has a length of up to approximately 15 nucleotides.

The second adapter oligonucleotide comprises an oligonucleotide sequence complementary to the second portion of the catcher oligonucleotide adjacent to an oligonucleotide sequence complementary to the second portion of the target nucleic acid. The oligonucleotide sequence complementary to the second portion of the catcher oligonucleotide preferably has a length of up to approximately 15 nucleotides. The oligonucleotide sequence complementary to the second portion of the target nucleic acid has preferably a length of up to approximately 15 nucleotides.

In a preferred embodiment, the first and/or the second adapter oligonucleotide is/are single-stranded adapter oligonucleotides.

In a preferred embodiment, the first and/or the second adapter oligonucleotide are oligodesoxyribonucleotides.

The first adapter oligonucleotide and/or the second adapter oligonucleotide have a label. The term "label" refers to any property of the adapter oligonucleotide that can be used for indicating the presence of the adapter oligonucleotide. The label is preferably a reporter molecule that is directly or indirectly attached to the adapter oligonucleotide and capable of producing a detectable signal. The reporter molecule is preferably an enzyme, more preferred an esterase, or a fluorescent dye. The reporter molecule can be attached to one of the two ends of the adapter oligonucleotide. The reporter molecule can also be attached inbetween the two ends of the adapter oligonucleotide, for example it can be linked to a base.

The catcher oligonucleotide hybridizes with the respective complementary portions of the first and the second adapter oligonucleotide and the target nucleic acid hybridizes with the respective complementary portions of the first and the second adapter oligonucleotide such that a Holliday junction is formed.

The term "hybridizing" refers to the formation of base pairs between complementary sequences of two single-stranded RNA or DNA molecules. Base pairing occurs by the formation of hydrogen bridges between complementary bases. The term "base pair" includes Watson-Crick base pairs as well as non-Watson-Crick base pairs such as the so-called wobble base pair. A typical wobble base pair is a base pair of guanine with uracil that may form during hybridzation of a DNA molecule with an RNA molecule.

In a preferred embodiment, the oligonucleotide sequence of the target nucleic acid is fully complementary to the respective complementary portions of the first and the second adapter oligonucleotide. Thus, the target nucleic acid fully hybridizes with the respective complementary portions of the adapter oligonucleotides. The term "fully hybridize" means that there is not a single base pair mismatch, i.e. each single base of the target nucleic acid forms a base pair with a complementary base of the first or the second adapter oligonucleotide. If the target nucleic acid is fully complementary to the respective complementary portions of the adapter oligonucleotides, the target nucleic acid can be identified and/or quantified with high specificity.

In a preferred embodiment, the oligonucleotide sequences of the first and the second portion of the catcher oligonucleotide are fully complementary to the respective complementary portions of the first and the second adapter oligonucleotide. This results in stable hybridization of the catcher oligonucleotide with the adapter oligonucleotides, increasing the reliability of identification and quantification of the target nucleic acid.

In a preferred embodiment, the first and the second adapter oligonucleotide and/or the catcher oligonucleotide are oligodesoxyribonucleotides. Oligodesoxyribonucleotides are more stable compared to oligoribonucleotides and rarely form secondary structures.

In a more preferred embodiment, the first and the second adapter oligonucleotide are oligodesoxyribonucleotides, while the target nucleic acid is an RNA. Upon hybridization RNA/DNA duplexes are formed that are thermodynamically very stable.

The term "reaction condition" refers to at least one parameter or a combination of parameters at which at least one of the steps of the method is performed. It is preferred that the parameters comprise a temperature, a salt concentration, an ionic strength, a pH value and/or an addition of reagents such as formamide. In a preferred embodiment, the reaction condition is suited for melting the base pairs between the catcher oligonucleotide, the first and second adapter oligonucleotide and the target nucleic acid.

The first reaction condition is preferably a temperature.

The first reaction condition is the reaction condition at which the catcher oligonucleotide hybridizes with the respective complementary portions of the first and the second adapter oligonucleotide and at which the target nucleic acid hybridizes with the respective complementary portions of the first and the second adapter oligonucleotide such that a Holliday junction is formed. The choice of the first reaction condition preferably depends on the sequence and the length of the catcher and adapter oligonucleotides and the target nucleic acid. The selection of the first reaction condition can further depend on whether the catcher and adapter oligonucleotides and/or the target nucleic acids are RNA and/or DNA molecules.

In a preferred embodiment, the first reaction condition comprises a temperature of 45°C.

Upon hybridization, the target nucleic acid, the first adapter oligonucleotide, the second adapter oligonucleotide and the catcher oligonucleotide form a Holliday junction. The term "Holliday junction" refers to a cruciform structure formed by four single strands of nucleic acids and is named after Robin Holliday who proposed the four-way nucleic acid junction in 1964. Holliday junctions are four-component hybrids that are very stable. They were first observed in yeast as an intermediate during homologous recombination and are highly conserved from prokaryotes to mammals. Since the target nucleic acid is a crucial component for the formation of the stable Holliday junction, the Holliday junction is formed only if the target nucleic acid is present.

The method according to the invention further comprises incubating the carrier at a second reaction condition such that the first and the second adapter oligonucleotide remain on the support only if the target nucleic acid is present. Since the target nucleic acid is a crucial component for the formation of the stable Holliday junction, the Holliday junction is not formed if the target nucleic acid is not present. In this case, the remaining three-component structure of the catcher oligonucleotide, the first adapter oligonucleotide and the second adapter oligonucleotide is a sandwich arrangement of two-component hybrids formed by the catcher oligonucleotide and the first and/or the second adapter oligonucleotide.

The second reaction condition is the reaction condition at which the base pairing of the two-component hybrids of the catcher oligonucleotide and the first and/or the second adapter oligonucleotide are melted. During melting, hydrogen bridges between the paired bases are abrogated. This leads to removal of the first and the second adapter oligonucleotide from the support at the second reaction condition if no target nucleic acid is present. The second reaction condition does not lead to melting the base pairing of the four-component hybrid of the Holliday junction that is formed in the presence of target nucleic acid. The molecular interactions holding the catcher oligonucleotide, the first and the second adapter oligonucleotide and the target nucleic acid nucleotides together in the Holliday junction are very strong. This causes an increased melting temperature of the Holliday junction compared to two-component hybrids of the catcher oligonucleotide and the first and/or the second adapter oligonucleotide. Thus, if the target nucleic acid is present, the first and the second adapter oligonucleotide remain on the support at the second reaction condition due to their participation in the stable Holliday junction.

In a preferred embodiment, the second reaction condition comprises a higher temperature and/or a lower ionic strength compared to the first reaction condition. This facilitates break-down of the hydrogen bridges between paired bases at the second reaction condition.

In a preferred embodiment the second reaction condition comprises a temperature of 55°C.

The method according to the invention further comprises detecting the label of the first and/or the second adapter oligonucleotide. The presence of the label indicates the presence of the target nucleic acid. Since the adapter oligonucleotides are present only if the target nucleic acid is present so that a Holliday junction is formed, detection of the label identifies the target nucleic acid. Label detection can also be used for quantifying the target nucleic acid.

The method according to the invention allows direct identification and/or quantification of the target nucleic acid. In particular, it is not necessary to chemically or enzymatically modify the target nucleic acid. Since certain target nucleic acids preferably undergo modifications, the quantification of modified target nucleic acids may lead to imprecise results. This is avoided by directly detecting the target nucleic acid. In addition, artefacts that may occur during modifying the target nucleic acid are eliminated.

The method according to the invention is very sensitive so that even low levels of the target nucleic acid can be detected. As a consequence, amplification of the target nucleic acid prior to its identification is dispensable. This avoids the incorporation of incorrect nucleotides in the target nucleic acid so that the specificity of the method is increased.

The Holliday junction is very stable, facilitating a robust and quantitatively evaluable detection of the label. In particular, Holliday junctions show an increased stability compared to two-component hybrids. Thus, detecting the label of the first and/or the second adapter oligonucleotide is not very temperature sensitive, allowing the temperature to be optimally adjusted to the type of label used. The same applies to reaction conditions other than the temperature. This leads to the high sensitivity of the method.

In a preferred embodiment, two or more steps of the method according to the invention are combined with each other. This allows efficient performance of the method.

In a preferred embodiment, washing of the support is performed prior to step d). The washing step facilitates the removal of adapter oligonucleotides that have detached from the catcher oligonucleotide from the support. In addition, nucleic acids that are not hybridized to adapter oligonucleotides are removed from the support. This avoids background signal during label detection. Thus, label detection is highly specific, leading to a high specificity of identification of the target nucleic acid.

In a preferred embodiment, the second reaction condition is a stringent reaction condition. The term "stringent reaction condition" refers to a reaction condition in which only a target nucleic acid that is fully hybridized with the respective complementary portions of the first and the second adapter oligonucleotide remains on the support. Nucleic acid molecules having one or more base pair mismatches to the respective complementary portions of the adapter oligonucleotides detach from the adapter oligonucleotides. By applying a stringent reaction condition prior to label detection, a high specificity of the method is achieved.

In a preferred embodiment, the target nucleic acid is an RNA, preferably a microRNA (miRNA). miRNAs are non-coding single-stranded RNAs with a length of approximately 17 to approximately 25 nucleotides that play a role in the post-transcriptional regulation of gene expression. miRNAs form base pairs with complementary sequences within mRNA molecules with a high specificity, thereby regulating the translation of the mRNA in the corresponding protein.

In another embodiment, the target nucleic acid is a single-stranded small interfering RNA (siRNA). Similar to miRNAs siRNAs can also participate in the post-transcriptional regulation of gene expression by specifically forming base pairs with complementary sequences within mRNA molecules to regulate the translation of the mRNA in the corresponding protein.

In a preferred embodiment, the first reaction condition comprises incubating the carrier at a temperature of 85°C to 95°C and subsequently cooling down the carrier to room temperature. This facilitates the formation of Holliday junctions. Cooling down is preferably performed at a slow rate to further promote Holliday junction formation.

In a preferred embodiment, the melting temperature of the Holliday junction formed in step b) is at least 10°C, preferably at least 20°C higher than the melting temperature of a double strand hybrid of the first adapter oligonucleotide and the catcher oligonucleotide and/or a double strand hybrid of the second adapter oligonucleotide and the catcher oligonucleotide. Two-component hybrids such as double strand hybrids of the first or the second adapter oligonucleotide and the catcher oligonucleotide have less complementary base pairs compared to the four-component hybrid of the Holliday junction. Therefore, the double strand hybrids have a lower melting temperature and thus a lower stability compared to the Holliday junction. A high difference between the melting temperature of the double strand hybrids and the melting temperature of the Holliday junction allows a stringent second reaction condition to ensure that all adapter oligonucleotides that are not participating in a Holliday junction are removed from the carrier before detecting the label. This increases the specificity of the method.

In a preferred embodiment, at least two different target nucleic acids, preferably three to 130 different target nucleic acids are identified simultaneously, wherein the Holliday junction formed in step b) has the same melting temperature for all target nucleic acids. It is further preferred that 32 different target nucleic acids are identified simultaneously on a common support.

For the parallel identification and/or quantification of multiple target nucleic acids (multiplexing), different first and second adapter oligonucleotides are used for each target nucleic acid. The adapter oligonucleotides differ at least in the oligonucleotide sequence that is complementary to the first or the second portion of the target nucleic acid.

The oligonucleotide sequence of the adapter oligonucleotides that is complementary to the first or the second portion of the target nucleic acid, the oligonucleotide sequence of the adapter oligonucleotides that is complementary to the first or the second portion of the catcher oligonucleotide and the the oligonucleotide sequence of the catcher oligonucleotide are selected such that the Holliday junction formed in step b) has the same melting temperature for all target nucleic acids. The term "same melting temperature" includes melting temperatures differing up to 4°C, preferably up to 2°C, from each other for different target nucleic acids. It is most preferred that the same melting temperature is an identical melting temperature.

The same melting temperature of all Holliday junctions allows performing step c) at a uniform second reaction condition for all nucleic acids. This enables the simultaneous identification and/or quantification of multiple target nucleic acids with high specificity on a common support.

In a preferred embodiment, the adapter oligonucleotides further differ in their label for each target nucleic acid. The different labels allow assigning of a signal obtained during label detection to a certain target nucleic acid.

In another embodiment, the detection of the label is performed position-specific in order to simultaneously identify and/or quantify different target nucleic acids. In this case, the same label can be used for all target nucleic acids. Position-specific label detection further allows to analyze a plurality of samples in one single method.

In a preferred embodiment, the label of the first and/or the second adapter oligonucleotide is an enzyme. The enzyme is preferably an esterase, more preferred a thermostable esterase. Due to its thermostability the enzymatic activity of the esterase is not affected by high temperatures during performance of the method. It is thus possible to contact the support with an enzyme-labelled adapter oligonucleotide already at the beginning of the method even though the label is only detected at the end of the method.

The esterase is preferably the thermostable esterase 2 of *Alicyclobacillus acidocaldarius.* Esterase 2 is formed by a single protein chain and can thus be easily coupled to the first and/or the second adapter oligonucleotide.

In another embodiment, the label is a fluorescent dye which is preferably covalently coupled to the first and/or the second adapter oligonucleotide. The use of fluorescent dyes facilitates simple and rapid label detection. In addition, no substrate is needed for detecting the label so that the method can be performed at low costs.

In a preferred embodiment, detecting the label is performed electrochemically. For electrochemical detection, substrates are reacted at the label, which is preferably an enzyme, to redox active reaction products. Thus, a current or a change in voltage can be measured at an electrode.

In another embodiment, detection of the label is performed at an assay temperature. The assay temperature is selected depending on the reporter molecule used. For example, if the reporter molecule is an enzyme, the assay temperature is in a range of high enzymatic activity of the enzyme. If esterase 2 of *Alicyclobacillus acidocaldarius* is used, the assay temperature is preferably around 35°C.

In another embodiment, detecting the label is performed optically. Optical detection of the label is simple and can be performed at low costs.

In a preferred embodiment, the first and/or the second adapter oligonucleotide comprises an adapter oligonucleotide extension. The adapter oligonucleotide extension is preferably used as the label of the adapter oligonucleotide. It is preferred that the adapter oligonucleotide extension comprises an oligonucleotide sequence complementary to at least a portion of a reporter conjugate. The reporter conjugate is an oligonucleotide that has at least one reporter molecule. Label detection is performed by hybridising the reporter conjugate with the adapter oligonucleotide extension.

In a more preferred embodiment, the adapter oligonucleotide extension comprises a universal oligonucleotide sequence that is used as the label of the adapter oligonucleotide. For example, the universal oligonucleotide sequence is an oligonucleotide sequence complementary to at least a portion of a reporter conjugate. The universal oligonucleotide sequence within the adapter oligonucleotide extension allows uniform label detection even if different first and/or second adapter oligonucleotides are used.

In a preferred embodiment, the adapter oligonucleotide extension is formed by a stable single-stranded nucleic acid, preferably by a viral RNA.

In a preferred embodiment, the label is detected by a branched DNA assay. The branched DNA assay is a signal amplification assay for detecting nucleic acid molecules. In short, the target nucleic acid is labelled by addition of a so-called label extender, the label extender hybridizes with a so-called pre-amplifier that in turn hybridises with amplifier molecules. Pre-amplifier and amplifier molecules are short single-stranded oligodesoxyribonucleotides. The amplifier molecules carry one or multiple reporter molecules. The amplifier molecules are branched DNA molecules allowing for very dense decoration of the pre-amplifier leading to a high sensitivity of the branched DNA assay. Thus, the combination of the method according to the invention with the branched DNA assay allows easy implementation of increased sensitivity. A further advantage of using the branched DNA assay for detecting the label of the first and/or the second adapter oligonucleotide is that the reagents needed to perform the assay are readily available.

The branched DNA assay can also be used for identifying different target nucleic acids in a multiplex setting. In this case, the specificity and the reliability of the method is preferably increased by combinatorial fluorescence.

In a more preferred embodiment, the first and/or the second adapter oligonucleotide comprises an adapter oligonucleotide extension, the adapter oligonucleotide extension comprising a first portion and a second portion adjacent to each other, and step d) comprises contacting the support with at least one first label extender, at least one second label extender and at least one pre-amplifier. The pre-amplifier comprises a first portion and a second portion adjacent to each other. The first label extender comprises an oligonucleotide sequence complementary to the first portion of the adapter oligonucleotide extension adjacent to an oligonucleotide sequence complementary to the first portion of the pre-amplifier. The second label extender comprises an oligonucleotide sequence complementary to the second portion of the adapter oligonucleotide extension adjacent to an oligonucleotide sequence complementary to the second portion of the pre-amplifier. The adapter oligonucleotide extension hybridizes with the respective complementary portions of the first and the second label extender and the pre-amplifier hybridizes with the respective complementary portions of the first and the second label extender such that the adapter oligonucleotide extension, the first label extender, the second label extender and the pre-amplifier form a reporter Holliday junction. For detection of the reporter Holliday junction, branched amplifier molecules carrying reporter molecules are added.

In a second aspect, the invention is directed to a kit for detecting at least one single-stranded target nucleic acid in a sample, the target nucleic acid comprising a first portion and a second portion adjacent to each other, the kit comprising at least one solid support with at least one catcher oligonucleotide immobilized on the support, the catcher oligonucleotide comprising a first portion and a second portion adjacent to each other, at least one first adapter oligonucleotide, the first adapter oligonucleotide comprising an oligonucleotide sequence complementary to the first portion of the catcher oligonucleotide adjacent to an oligonucleotide sequence complementary to the first portion of the target nucleic acid, and at least one second adapter oligonucleotide, the second adapter oligonucleotide comprising an oligonucleotide sequence complementary to the second portion of the catcher oligonucleotide adjacent to an oligonucleotide sequence complementary to the second portion of the target nucleic acid, the first and/or the second adapter oligonucleotide having a label, wherein the label of the first and/or the second adapter oligonucleotide indicates the presence of the target nucleic acid in the sample.

The sample can be a mixture of the target nucleic acid with other nucleic acids and/or proteins. The sample can further be a lysate that is preferably isolated from human, animal, plant or bacterial cells. In a preferred embodiment, the cells are derived from a biopsy of a patient. If the patient has a disease or is suspected of having a disease in which the target nucleic acid is specifically increased or decreased, the kit can be used for diagnostic detection of the target nucleic acid in the biopsy. In a preferred embodiment, the disease is cancer, more preferred breast cancer, lung cancer or leukaemia. In another embodiment, the disease is a malfunction of the immune system.

In a preferred embodiment, the label is an enzyme and the kit further comprises at least one substrate that is specific for the enzyme.

In a preferred embodiment, the target nucleic acid is an RNA, preferably a miRNA.

In the following preferred embodiments of the invention are explained with reference to the drawings.

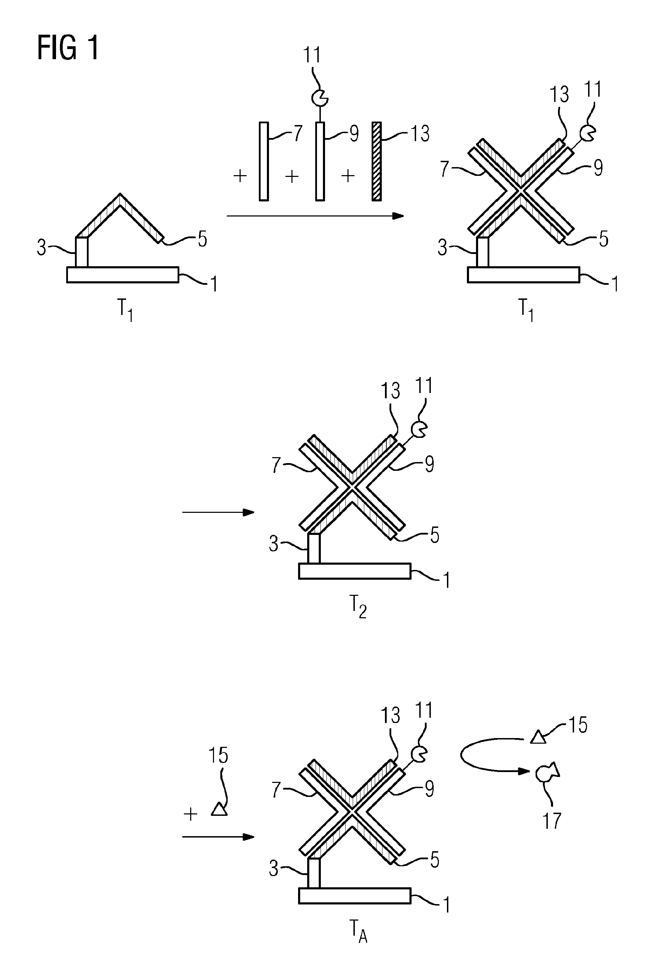

Figure 1 shows the identification of a single-stranded target nucleic acid 13 using the method according to the invention. The target nucleic acid 13 is miR-16. MiR-16 is a miRNA that can reduce the expression of the anti-apoptotic protein Bcl-2 in lymphocytes. A silicon chip with two integrated gold electrodes is used as a solid support 1. A single-stranded catcher oligonucleotide 5 is immobilized on the chip via a spacer 3. First, the chip is contacted with the miR-16, a first adapter oligonucleotide 7 and a second adapter oligonucleotide 9 at a first temperature of 45°C. The second adapter oligonucleotide 9 is labelled with a reporter 11. The reporter 11 is a thermostable esterase 2 of *Alicyclobacillus acidocaldarius* that is covalently coupled to the second adapter oligonucleotide 9. The catcher oligonucleotide 5 comprises two oligonucleotide sequences located directly adjacent to each other, namely a first portion and a second portion. The first portion of the catcher oligonucleotide 5 is fully complementary to an oligonucleotide sequence of the first adapter oligonucleotide 7 while the second portion of the catcher oligonucleotide 5 is fully complementary to an oligonucleotide sequence of the second adapter oligonucleotide 9. The first adapter oligonucleotide 7 comprises an oligonucleotide sequence fully complementary to the first portion of the catcher oligonucleotide 5 adjacent to an oligonucleotide sequence fully complementary to a first portion of miR-16. The first portion of miR-16 is directly adjacent to a second portion of miR-16. The second adapter oligonucleotide 9 comprises an oligonucleotide sequence fully complementary to the second portion of the catcher oligonucleotide 5 directly adjacent to an oligonucleotide sequence fully complementary to the second portion of miR-16. The chip is incubated for 2 minutes at 90°C and subsequently cooled down to room temperature. During cool-down, the catcher oligonucleotide 5 and miR-16 hybridize with the respective complementary portions of the first and the second adapter oligonucleotide 7, 9. As a result, the catcher oligonucleotide 5, the first adapter oligonucleotide 7, the second adapter oligonucleotide 9 and miR-16 form a Holliday junction. The chip is now incubated at a second temperature of 55°C for 10 minutes. Due to the presence of miR-16, the Holliday junction remains stable. The chip is then brought to an assay temperature of 35°C at which esterase 2 is detected. Esterase 2 is read out electrochemically. To do so, p-aminophenylbutyrate, a substrate 15 of esterase 2, is added to the chip. Esterase 2 reacts p-aminophenylbutyrate to a redox active reaction product 17 p-aminophenol. Redox cycling of p-aminophenol and quinonimine generates a current that is measured at the gold electrode. Measurement of the current indicates the presence of esterase 2 that indicates the presence of miR-16.

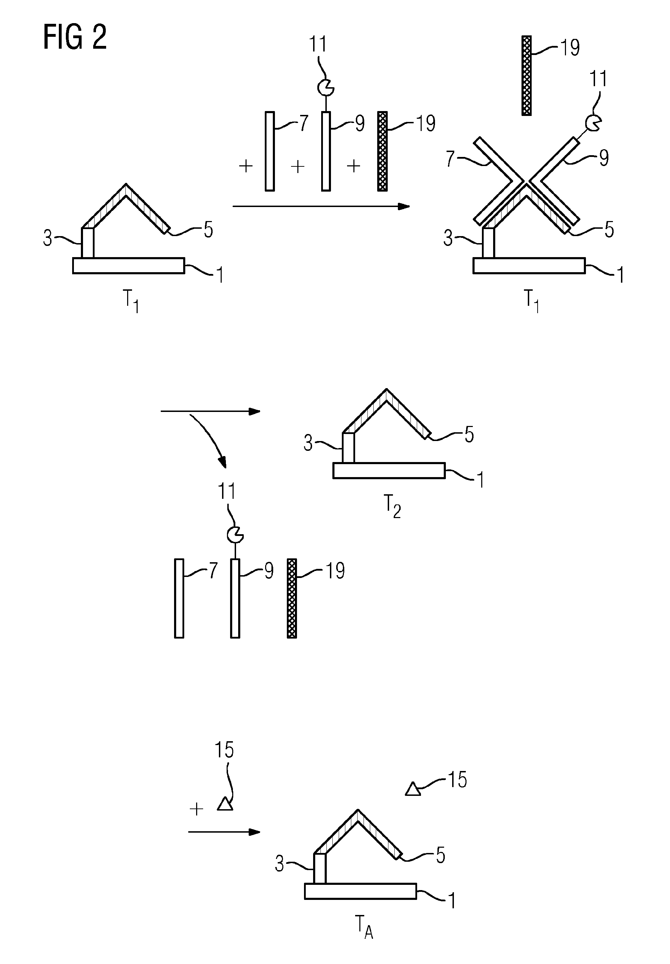

Figure 2 shows the method according to the invention in case a nucleic acid 19 whose sequence is not identical to the sequence of the target nucleic acid 13 is present. The nucleic acid 19 is used as a negative control for verifying the method according to the invention. Figure 2 shows that the nucleic acid 19 does not hybridise with the respective portions of the first and the second adapter oligonucleotide 7, 9 while the chip is incubated for 2 minutes at 90°C and subsequently cooled down to room temperature. Thus, no stable Holliday junction is formed. During incubation of the chip at 55°C for 10 minutes the base-pairs formed between the catcher oligonucleotide 5 and the respective complementary portions of the first and the second adapter oligonucleotide 7, 9 melt. Thus, the first and the second adapter oligonucleotide 7, 9 as well as the nucleic acid 19 are removed from the chip. Since no esterase 2 remains on the chip, no current is measured during the detection of the esterase.

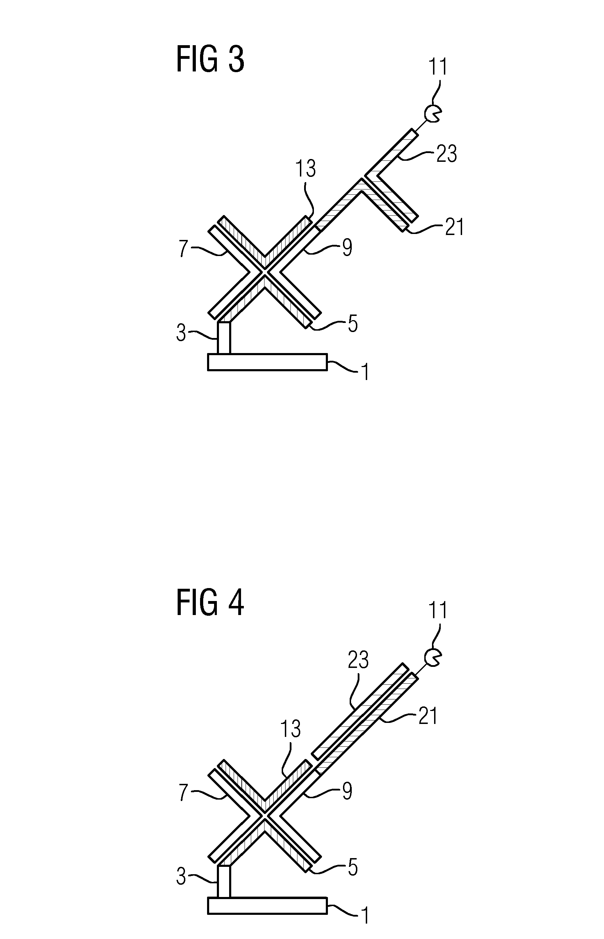

Figure 3 shows a preferred embodiment of the method according to the invention. In this embodiment, the label of the second adapter oligonucleotide 9 is formed by an adapter oligonucleotide extension 21. The adapter oligonucleotide extension 21 has a universal nucleotide sequence comprising an oligonucleotide sequence fully complementary to a portion of a reporter conjugate 23. A reporter 11 is covalently bound to the reporter conjugate 23. The reporter 11 is esterase 2 of *Alicyclobacillus acidocaldarius.* The reporter conjugate 23 is hybridized to the respective complementary sequence of the adapter oligonucleotide extension 21. The presence of the reporter conjugate 23 indicating the presence of miR-16 is detected by reading out esterase 2.

Figure 4 shows another preferred embodiment of the method according to the invention. The difference to the embodiment shown in Figure 3 is that the sequence of the adapter oligonucleotide extension 21 is fully complementary to the sequence of the reporter conjugate 23. As shown in Figure 4, the reporter conjugate 23 and the adapter oligonucleotide extension 21 hybridize with each other over their full length. As a result, one end of the reporter conjugate 23 is adjacent to the second portion of miR-16. The presence of the reporter conjugate 23 indicating the presence of miR-16 is detected by reading out esterase 2.

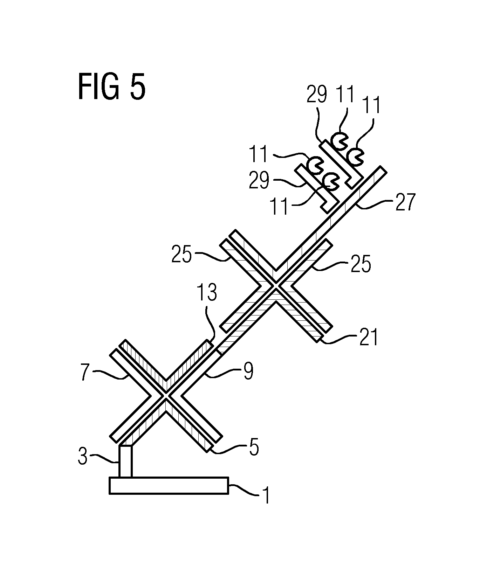

Figure 5 shows a preferred embodiment of the method according to the invention in which the presence of miR-16 is detected by a branched DNA assay. The second adapter oligonucleotide 9 comprises an adapter oligonucleotide extension 21. The adapter oligonucleotide extension 21 comprises two oligonucleotide sequences that are complementary to a portion of a label extender 25. The chip is incubated with two label extenders 25, each comprising a portion that hybridises with the respective complementary oligonucleotide sequence of the adapter oligonucleotide extension 21. These portions of the label extenders 25 are directly adjacent to portions of the label extenders 25 that are subsequently hybridised with complementary oligonucleotide sequences within a pre-amplifier 27. As a result, the adapter oligonucleotide extension 21, the two label extenders 25 and the pre-amplifier 27 form a reporter Holliday junction, leading to the presence of two Holliday junctions on the chip. The first Holliday junction is formed by the catcher oligonucleotide 5, the first adapter oligonucleotide 7, the second adapter oligonucleotide 9 and miR-16. The second Holliday junction is the reporter Holliday junction formed by the adapter oligonucleotide extension 21, the two label extenders 25 and the pre-amplifier 27. For detecting the presence of miR-16, the pre-amplifier 27 is hybridised with multiple branched amplifiers 29 having multiple reporters 11 that are then read-out.

## Claims

1. A method for identifying and quantifying at least one single-stranded target nucleic acid (13), comprising the steps of:
a) providing at least one solid support (1) with at least one catcher oligonucleotide (5) immobilized on the support (1), the catcher oligonucleotide (5) comprising a first portion and a second portion adjacent to each other;
b) contacting the support (1) at a first reaction condition with at least one first adapter oligonucleotide (7), at least one second adapter oligonucleotide (9) and at least one single-stranded target nucleic acid (13), the target nucleic acid (13) comprising a first portion and a second portion adjacent to each other, the first adapter oligonucleotide (7) comprising an oligonucleotide sequence complementary to the first portion of the catcher oligonucleotide (5) adjacent to an oligonucleotide sequence complementary to the first portion of the target nucleic acid (13), the second adapter oligonucleotide (9) comprising an oligonucleotide sequence complementary to the second portion of the catcher oligonucleotide (5) adjacent to an oligonucleotide sequence complementary to the second portion of the target nucleic acid (13), the first and/or the second adapter oligonucleotide (7, 9) having a label, the catcher oligonucleotide (5) hybridizes with the respective complementary portions of the first and the second adapter oligonucleotide (7, 9) and the target nucleic acid (13) hybridizes with the respective complementary portions of the first and the second adapter oligonucleotide (7, 9) such that a Holliday junction is formed;
c) incubating the carrier (1) at a second reaction condition such that the first and the second adapter oligonucleotide (7, 9) remain on the support only if the target nucleic acid (13) is present;
d) detecting the label of the first and/or the second adapter oligonucleotide (7, 9).

2. The method of claim 1, **characterised in that** the first reaction condition comprises incubating the carrier (1) at a temperature of 85°C to 95°C and subsequently cooling down the carrier (1) to room temperature.

3. The method of claim 1 or 2, **characterised in that** the melting temperature of the Holliday junction formed in step b) is at least 10°C, preferably at least 20°C higher than the melting temperature of a double strand hybrid of the first adapter oligonucleotide (7)and the catcher oligonucleotide (5) and/or a double strand hybrid of the second adapter oligonucleotide (9) and the catcher oligonucleotide (5).

4. The method of any of claims 1 to 3, **characterised in that** at least two different target nucleic acids (13), preferably three to 130 different target nucleic acids (13) are identified simultaneously, wherein the Holliday junction formed in step b) has the same melting temperature for all target nucleic acids (13).

5. The method of any of claims 1 to 4, **characterised in that** the label is detected by a branched DNA assay.

6. The method of claim 5, **characterised in that** the first and/or the second adapter oligonucleotide (7, 9) comprises an adapter oligonucleotide extension (21), the adapter oligonucleotide extension (21) comprising a first portion and a second portion adjacent to each other, and step d) comprises contacting the support (1) with at least one first label extender, at least one second label extender and at least one pre-amplifier (27), the pre-amplifier (27) comprising a first portion and a second portion adjacent to each other, the first label extender comprises an oligonucleotide sequence complementary to the first portion of the adapter oligonucleotide extension (21) adjacent to an oligonucleotide sequence complementary to the first portion of the pre-amplifier (27), the second label extender comprises an oligonucleotide sequence complementary to the second portion of the adapter oligonucleotide extension (21) adjacent to an oligonucleotide sequence complementary to the second portion of the pre-amplifier (27), the adapter oligonucleotide extension (21) hybridizes with the respective complementary portions of the first and the second label extender and the pre-amplifier (27) hybridizes with the respective complementary portions of the first and the second label extender such that the adapter oligonucleotide extension (21), the first label extender, the second label extender and the pre-amplifier (27) form a reporter Holliday junction.

7. The method of any of claims 1 to 6, **characterised in that** the target nucleic acid (13) is an RNA, preferably a miRNA.

8. A kit for detecting at least one single-stranded target nucleic acid (13) in a sample, the target nucleic acid (13) comprising a first portion and a second portion adjacent to each other, the kit comprising
at least one solid support (1) with at least one catcher oligonucleotide (5) immobilized on the support (1), the catcher oligonucleotide (5) comprising a first portion and a second portion adjacent to each other;
at least one first adapter oligonucleotide (7), the first adapter oligonucleotide (7) comprising an oligonucleotide sequence complementary to the first portion of the catcher oligonucleotide (5) adjacent to an oligonucleotide sequence complementary to the first portion of the target nucleic acid (13); and
at least one second adapter oligonucleotide (9), the second adapter oligonucleotide (9) comprising an oligonucleotide sequence complementary to the second portion of the catcher oligonucleotide (5) adjacent to an oligonucleotide sequence complementary to the second portion of the target nucleic acid (13), the first and/or the second adapter oligonucleotide (7, 9) having a label;
wherein the label of the first and/or the second adapter oligonucleotide (7, 9) indicates the presence of the target nucleic acid (13) in the sample.
